# DEMANDE DE BREVET EUROPEEN

(11) **EP 3 812 051 A1**
(43) Date de publication de la demande: **28.04.2021**
(21) Numéro de dépôt: 20202129.1
(22) Date de dépôt: 15.10.2020
(51) Int. Cl.: B07B 1/26, A61K 36/185

(54) **PROCEDE ET DISPOSITIF DE SEPARATION DES TRICHOMES DU CHANVRE**

(30) Priorité: 21.10.2019 FR 1911766
(71) Demandeur: ELICAN BIOTECH, 59120 Loos (FR)
(72) Inventeur: MEXMAIN, Gérald, 59560 COMINES (FR)
(74) Mandataire: Plasseraud IP

(57) **Abrégé**

Procédé de séparation des trichomes de plantes de chanvre comprenant les étapes :
a) fourniture d'un dispositif de séparation (1) comprenant :
- un bâti (2),
- au moins un tambour (3) monté rotatif par rapport au bâti (2), dont la paroi périphérique (31) est constituée d'un tamis (32),
- un circuit de recirculation et de tri (4),

b) mise en rotation du tambour (3) par rapport au bâti (2),
c) insertion de pellets de glace carbonique dans le tambour (3),
d) insertion de plantes de chanvre dans le tambour (3),
e) réception de la matière en sortie du tambour (3),
f) tri de la matière réceptionnée en sortie du tambour (3) puis amenée des pellets de glace carbonique en entrée du tambour (3),
g) évacuation des trichomes,
dans lequel les étapes b) à g) sont effectuées simultanément et en continu au cours du procédé.

## Description

L'invention concerne un procédé et un dispositif de séparation des trichomes du chanvre.

### Domaine technique

L'invention relève du domaine de l'extraction des trichomes du chanvre à séché ou frais, et notamment en vue d'une utilisation thérapeutique des substances actives du chanvre, présentes dans lesdits trichomes.

### Technique antérieure

Les plantes de chanvre se présentent sous la forme de tiges avec une pluralité de feuilles ainsi que des fleurs. Durant la floraison de la plante, des petites glandes de résine, situées sur des poils d'accroche, apparaissent sur les fleurs et sur les feuilles de la plante, connues sous le nom de trichomes, et à l'intérieur desquels se trouvent les substances actives du chanvre, comme par exemple le cannabigérol (CBG), le cannabidol (CBD), le tetrahydrocannbidiol (THC), etc.

Il peut être souhaitable de récupérer ces substances actives, notamment à des fins d'utilisation thérapeutique, par exemple pour la fabrication de médicaments, tels que décrit dans les préconisations de l'Agence Nationale de Sécurité du Médicament et des Produits de Santé (ANSM), établissement public Français ayant pour mission principale d'évaluer les risques sanitaires présentés par les médicaments et produits de santé destinés à l'être humain.

L'ANSM préconise notamment l'emploi de substances actives du chanvre en cas de soulagement insuffisant ou d'une mauvaise tolérance (effets indésirables) des thérapeutiques médicamenteuses ou non accessibles, en tenant compte des recommandations de bonnes pratiques pour :
- les douleurs neuropathiques réfractaires aux thérapies (médicamenteuses ou non) accessibles,
- certaines formes d'épilepsie pharmacorésistantes,
- certains symptômes rebelles en oncologie (tels que nausées, vomissements, anorexie...),
- les situations palliatives,
- la spasticité douloureuse de la sclérose en plaques ou des autres pathologies du système nerveux central.

Afin de récupérer ces substances actives, il est souhaitable de les extraire de la plante de chanvre, ce qui notamment par une séparation des trichomes du reste de la plante (tige, feuilles et fleurs).

A cette fin, il est connu diverses techniques permettant l'extraction des substances actives de la plante du chanvre, comme par exemple par la dissolution des trichomes dans du solvant pour l'isoler du reste de la plante par une filtration ultérieure.

On connaît également la séparation des trichomes du reste de la plante par action mécanique qui consiste à secouer la plante de chanvre pour que les trichomes s'en détachent puis à les séparer du reste de la plante, grâce à l'emploi d'un tamis.

Afin de faciliter le détachement des trichomes, en rendant les poils d'accroche plus cassants, il peut être prévu de refroidir la plante de chanvre, notamment à une température négative, préalablement ou simultanément à l'action mécanique exercée sur celle-ci.

On connait ainsi du document EP 1 329 265 A2 un procédé et un dispositif de séparation des trichomes de la plante de chanvre. Le dispositif prévoit un tambour rotatif dont la paroi périphérique est constituée par un tamis métallique avec des ouvertures comprises entre 0,02 et 0, 05 mm², à l'intérieur duquel sont introduits des plantes de chanvre, préalablement séchées, et dont les tiges et les graines ont été ôtées. Le tambour est ensuite mis en rotation pendant 20 à 30 minutes et le frottement des plantes de chanvres contre la paroi périphérique du tambour permet le détachement des trichomes de la plante de chanvre et leur passage au travers des ouvertures du tamis afin d'être récupérés dans un compartiment séparé.

En fonctionnement, les plantes de chanvre peuvent être refroidies à une température comprise entre 0 et -5°C afin de faciliter la séparation des trichomes de la plante du chanvre.

Afin de produire des chocs sur les plantes de chanvre à l'intérieur du tambour, il peut être prévu des barres d'entretoisement à l'intérieur du tambour disposées à distance de la paroi périphérique du tambour, vers l'axe de rotation du tambour.

Un tel dispositif et le procédé de séparation des trichomes de la plante de chanvre qui y est mis en œuvre présentent néanmoins plusieurs inconvénients.

Tout d'abord, le temps de séjour de la plante de chanvre dans le tambour d'une durée de 20 à 30 minutes induit un fonctionnement discontinu du dispositif de séparation et du procédé qui y est mis en œuvre, et nécessite l'arrêt du tambour pour en extraire la matière traitée et y introduire de la matière à traiter (et éventuellement collecter les trichomes séparés de la plante de chanvre), ce qui réduit considérablement la capacité de production d'un tel dispositif et d'un tel procédé, les rendant difficilement compatible pour une application à l'échelle industrielle, prévoyant par exemple le traitement de plusieurs centaines de kilogrammes, voire plusieurs tonnes de chanvre par jour.

Également, pour obtenir une séparation optimale des trichomes de la plante de chanvre avec un tel dispositif de séparation et le procédé qui y est mis en œuvre, il est nécessaire de sécher préalablement les plantes de chanvre et d'ôter les tiges et les graines, ce qui augmente considérablement la durée entre la récolte de la plante de chanvre et la récupération des trichomes, et augmente également le coût de revient global.

### Problème technique

L'invention vient pallier ces inconvénients et les autres de l'art antérieur en proposant un procédé de séparation des trichomes de la plante de chanvre et un dispositif pour la mise en œuvre de ce procédé d'une capacité de production augmentée compatible avec une application à une échelle industrielle, prévoyant notamment le traitement de plusieurs centaines de kilogrammes, voire de plusieurs tonnes de plantes de chanvre par jour.

Un autre but de la présente invention est de proposer un tel procédé de séparation et un tel dispositif le mettant en œuvre permettant de réduire sensiblement la durée entre la récolte du chanvre et la récupération des trichomes.

Un autre but de la présente invention est de proposer un tel procédé de séparation et un tel dispositif le mettant en œuvre avec un coût de revient réduit.

Il est proposé un procédé de séparation des trichomes de plantes de chanvre comprenant les étapes :
a) fourniture d'un dispositif de séparation des trichomes de plantes de chanvre comprenant :
   - un bâti,
   - au moins un tambour monté rotatif par rapport au bâti autour de son axe longitudinal, la paroi périphérique du tambour étant constituée, au moins partiellement, d'un tamis, ledit tambour comprenant une ouverture au niveau de chacune de ses extrémités longitudinales matérialisant respectivement une entrée et une sortie pour la matière circulant dans ledit tambour, le tambour étant disposé sur le bâti de sorte que son axe longitudinal soit, selon une première alternative : incliné par rapport à l'Horizontale avec un angle non nul, de telle sorte que l'ouverture d'entrée soit située au-dessus de l'ouverture de sortie selon la Verticale, ou selon une deuxième alternative : incliné sensiblement selon l'Horizontale, de telle sorte que l'ouverture d'entrée soit située sensiblement au même niveau que l'ouverture de sortie selon la verticale,
   - un circuit de recirculation et de tri, configuré pour recevoir de la matière en sortie du tambour, la trier puis l'amener, au moins en partie, en entrée du tambour,
   - au moins un compartiment de réception des trichomes configuré de sorte à réceptionner les trichomes passés à travers le tamis de la paroi périphérique du tambour, après leur séparation du reste de la plante de chanvre dans ledit tambour et à permettre leur évacuation du dispositif de séparation par l'intermédiaire de moyens d'évacuation,
b) mise en rotation du tambour par rapport au bâti autour de son axe longitudinal,
c) insertion de pellets de glace carbonique dans le tambour au travers de l'ouverture d'entrée du tambour,
d) insertion de plantes de chanvre dans le tambour au travers de l'ouverture d'entrée du tambour,
e) réception de la matière au niveau de l'ouverture de sortie du tambour comprenant au moins des résidus de plantes de chanvre mélangés aux pellets de glace carbonique,
f) tri de la matière réceptionnée au niveau de l'ouverture de sortie du tambour afin de séparer les résidus de plantes de chanvre des pellets de glace carbonique puis amenée des pellets de glace carbonique au niveau de l'ouverture d'entrée du tambour,
g) évacuation des trichomes du au moins un compartiment de réception des trichomes par l'intermédiaire des moyens d'évacuation.

Selon l'invention, les étapes b) à g) sont effectuées simultanément et en continu au cours du procédé.

Selon des caractéristiques optionnelles de l'invention, prises seules ou en combinaison :
- le dispositif de séparation comprend en outre des moyens de mise en vibration de la paroi périphérique du tambour, configurés pour mettre en vibration la paroi périphérique du tambour de façon continue au cours des étapes b) à g) du procédé, et, de façon continue au cours du procédé, le tamis de la paroi périphérique du tambour est décolmaté par la mise en vibration de la paroi périphérique du tambour ;
- les moyens de mise en vibration comprennent des billes de plastique configurées de sorte à venir percuter la paroi périphérique du tambour au cours de sa rotation autour de son axe longitudinal afin d'entraîner sa mise en vibration,
   le procédé comprenant en outre une étape b'), simultanée avec les étapes b) à g) et continue au cours du procédé, d'introduction de billes de plastique dans le tambour au travers de l'ouverture d'entrée du tambour, et :
   -- au cours de l'étape e) les billes sont réceptionnées au niveau de l'ouverture de sortie du tambour mélangées avec les résidus de plantes de chanvre et les pellets de glace carbonique, et
   -- au cours de l'étape f) les billes sont séparées des résidus de plantes de chanvre et amenées au niveau de l'ouverture d'entrée du tambour ;
- les pellets de glace carbonique possèdent une température comprise entre -50°C et -90°C, de préférence entre -60°C et -80°C, lorsque introduits dans le tambour au travers de l'ouverture d'entrée du tambour au cours de l'étape c) ;
- le dispositif de séparation comporte deux tambours positionnés l'un au-dessus de l'autre selon la Verticale avec un tambour supérieur et un tambour inférieur, le dispositif comprenant en outre une gouttière de communication entre le tambour supérieur et le tambour inférieur, configurée de sorte à acheminer la matière au niveau de l'ouverture de sortie du tambour supérieur vers l'ouverture d'entrée du tambour inférieur, les plantes de chanvre et les pellets de glace étant insérés au travers de l'ouverture d'entrée du tambour supérieur au cours des étapes c) et d) et les résidus de plantes de chanvre mélangés aux pellets de glace carbonique étant réceptionnés au niveau de l'ouverture de sortie du tambour inférieur au cours de l'étape e).

Selon un autre aspect, il est proposé un dispositif de séparation des trichomes de plantes de chanvre configuré pour permettre la mise en œuvre du procédé selon l'une des revendications 1 à 6, comprenant :
- un bâti,
- au moins un tambour monté rotatif par rapport au bâti autour de son axe longitudinal, la paroi périphérique du tambour étant constituée, au moins partiellement, d'un tamis ledit tambour comprenant une ouverture au niveau de chacune de ses extrémités longitudinales matérialisant respectivement une entrée et une sortie pour la matière circulant dans ledit tambour, le tambour étant disposé sur le bâti de sorte que son axe longitudinal soit, selon une première alternative : incliné par rapport à l'Horizontale avec un angle non nul, de telle sorte que l'ouverture d'entrée est située au-dessus de l'ouverture de sortie selon la Verticale, ou selon une deuxième alternative : incliné sensiblement selon l'Horizontale, de telle sorte que l'ouverture d'entrée soit située sensiblement au même niveau que l'ouverture de sortie selon la verticale,
- un circuit de recirculation et de tri, configuré pour recevoir de la matière en sortie du tambour, la trier puis l'amener, au moins en partie, en entrée du tambour,
- au moins un compartiment de réception des trichomes configuré de sorte à réceptionner les trichomes passés à travers le tamis de la paroi périphérique du tambour, après leur séparation du reste de la plante de chanvre dans ledit tambour et à permettre leur évacuation du dispositif de séparation par l'intermédiaire de moyens d'évacuation,
- préalablement à l'étape d) d'insertion de plantes de chanvre dans le tambour au travers de l'ouverture d'entrée du tambour, les plantes de chanvre sont convoyées et refroidies simultanément préalablement à leur introduction au travers de l'ouverture d'entrée du tambour au cours de ladite étape d).

Selon des caractéristiques optionnelles du dispositif de séparation, prises seules ou en combinaison :
- le dispositif de séparation est configuré de sorte à mettre en œuvre le procédé selon l'un des modes de réalisation de l'invention prévoyant que le dispositif de séparation comprend en outre des moyens de mise en vibration de la paroi périphérique du tambour, configurés pour mettre en vibration la paroi périphérique du tambour de façon continue au cours des étapes b) à g) du procédé, et que, de façon continue au cours du procédé, le tamis de la paroi périphérique du tambour est décolmaté par la mise en vibration de la paroi périphérique du tambour, le dispositif comprenant en outre des moyens de mise en vibration de la paroi périphérique du tambour ;
- le dispositif comporte au moins une piste de guidage entourant la paroi périphérique du tambour et au moins un galet de guidage, monté rotatif sur le bâti, coopérant avec ladite piste de guidage de sorte à assurer le guidage en rotation du tambour par rapport au bâti lors de sa rotation autour de son axe longitudinal, ladite piste de guidage comportant au moins un plot ménagé en saillie radiale vers l'extérieur du tambour, configuré de sorte que les contacts successifs entre le galet et ledit plot au cours de la rotation du tambour autour de son axe longitudinal entraînent la mise en vibration de la paroi périphérique du tambour ;
- le au moins un compartiment de réception des trichomes est solidaire du bâti et est positionné en dessous du tambour selon la Verticale, en regard du tamis, ledit compartiment de réception présentant une forme sensiblement pyramidale pointant vers le bas du tambour selon la Verticale, une ouverture d'évacuation étant prévue au niveau de la pointe de la pyramide formée par le compartiment de réception ;
- le dispositif présente au moins deux compartiments de réception, alignés selon l'axe longitudinal du tambour, configurés de sorte à collecter les trichomes sur toute la longueur du tambour ;
- les moyens d'évacuation comprennent un système d'aspiration muni d'un tuyau d'aspiration dont une extrémité est fixée au niveau de l'ouverture d'évacuation du compartiment de réception des trichomes ;
- le dispositif comprend deux tambours positionnés l'un au-dessus de l'autre selon la Verticale avec un tambour supérieur et un tambour inférieur, le dispositif comprenant en outre une gouttière de communication entre le tambour supérieur et le tambour inférieur, configurée de sorte à acheminer la matière au niveau de l'ouverture de sortie du tambour supérieur vers l'ouverture d'entrée du tambour inférieur ;
- le bâti et ledit au moins un tambour sont configurés de sorte à être disposés intégralement sur une remorque ;
- le circuit de recirculation et de tri comporte :
   -- un système de réception et de tri, disposé au niveau de l'ouverture de sortie du tambour, configuré de sorte à réceptionner la matière en sortie du tambour et à séparer les résidus de plantes de chanvre mélangés aux pellets de glace carbonique,
   -- un moyen de convoyage reliant ledit système de réception et de tri à l'ouverture d'entrée du tambour et configuré de sorte à convoyer les pellets de glace carbonique, après leur séparation des résidus de plante de chanvre au niveau du système de réception et de tri, jusqu'à l'ouverture d'entrée du tambour,
   -- des moyens d'évacuation des résidus de plante de chanvre, après leur séparation des pellets de glace carbonique au niveau du système de réception et de tri ;
- le système de réception et de tri comporte un compartiment de réception vibrant comprenant :
   -- un bac de réception présentant une paroi inférieure sensiblement plane avec une pluralité d'ouvertures sensiblement rectilignes,
   -- une paroi de support sensiblement plane avec une pluralité d'ouvertures sensiblement rectilignes, recevant en appui plan la paroi inférieure du bac de réception, la paroi inférieure du bac de réception étant mobile en translation par rapport à la paroi de support selon le plan d'appui entre ladite paroi inférieure du bac de réception et la paroi de support, avec une course limitée, la surface des ouvertures de la paroi inférieure du bac de réception étant strictement inférieure à la surface des ouvertures de la paroi de support et les ouvertures de la paroi inférieure du bac de réception étant inclinées par rapport aux ouvertures de la paroi de support, selon un angle non nul, le compartiment de réception vibrant étant configuré de sorte que sa mise en vibration entraîne un déplacement relatif entre la paroi inférieure du bac de réception et la paroi de support selon ledit plan d'appui de sorte à entraîner le passage au travers des ouvertures de la paroi inférieure du bac de réception et des ouvertures de la paroi de support des pellets de glace carbonique, en vue d'être convoyés jusqu'à l'ouverture d'entrée du tambour par l'intermédiaire du moyen de convoyage, tout en empêchant le passage des résidus de plante de chanvre au travers desdites ouvertures, de sorte à les maintenir dans ledit bac de réception, en vue d'être évacués par l'intermédiaire desdits moyens d'évacuation ;
- le moyen de convoyage comporte un tapis roulant présentant une première extrémité longitudinale et une deuxième extrémité longitudinale, avec :
   -- ladite première extrémité longitudinale reliée audit système de réception et de tri, et
   -- ladite deuxième extrémité longitudinale positionnée au niveau de l'ouverture d'entrée du tambour,
      dans lequel ledit tapis roulant est logé, au moins sur toute sa longueur entre ladite première extrémité longitudinale et ladite deuxième extrémité longitudinale, intégralement dans un carter, et un système de refroidissement est prévu, configuré de sorte à maintenir la température négative à l'intérieur du carter ;
- ledit système de réception et de tri comporte une virole de forme sensiblement cylindrique, solidaire du tambour, et sensiblement alignée avec ledit tambour, dans lequel ladite virole comprend une première extrémité longitudinale au niveau de l'ouverture de sortie du tambour, et une deuxième extrémité longitudinale présentant une ouverture d'évacuation, ladite virole présentant une paroi périphérique perforée, avec :
   -- sur une première portion longitudinale de la paroi périphérique , s'étendant depuis la première extrémité longitudinale de la virole, une pluralités d'ouvertures de diamètre compris entre 0,5 mm et 3 mm, de préférence entre 1,5 mm et 2,5 mm,
   -- sur une deuxième portion longitudinale de la paroi périphérique, s'étendant depuis la deuxième extrémité longitudinale de la virole, une pluralités d'ouvertures de diamètre compris entre 3,5 mm et 7 mm, de préférence entre 4mm et 6mm, ledit système de réception et de tri comprenant également une goulotte inclinée, reliant l'ouverture d'évacuation au moyen de convoyage, ladite goulotte comprenant une pluralité de tiges, disposées sensiblement parallèlement entre elles et écartées les unes des autres, avec une distance de séparation entre deux tiges consécutives sensiblement constante.

Les caractéristiques exposées dans les paragraphes suivants peuvent, optionnellement, être mises en œuvre. Elles peuvent être mises en œuvre indépendamment les unes des autres ou en combinaison les unes avec les autres.

### Brève description des dessins

D'autres caractéristiques, détails et avantages de l'invention apparaîtront à la lecture de la description détaillée ci-après, et à l'analyse des dessins annexés, sur lesquels :
**Fig. 1**

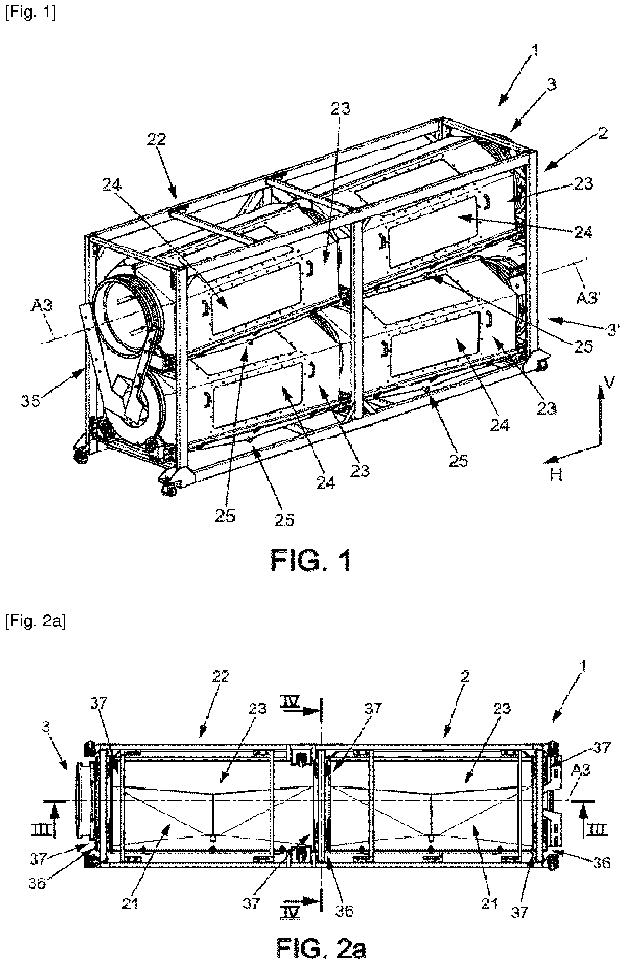

   [Fig. 1] montre une vue en perspective d'un dispositif de séparation selon un mode de réalisation conforme à l'invention.
**Fig. 2a**
   [Fig. 2a] montre une vue de dessous du dispositif de la figure 1.
**Fig. 2b**

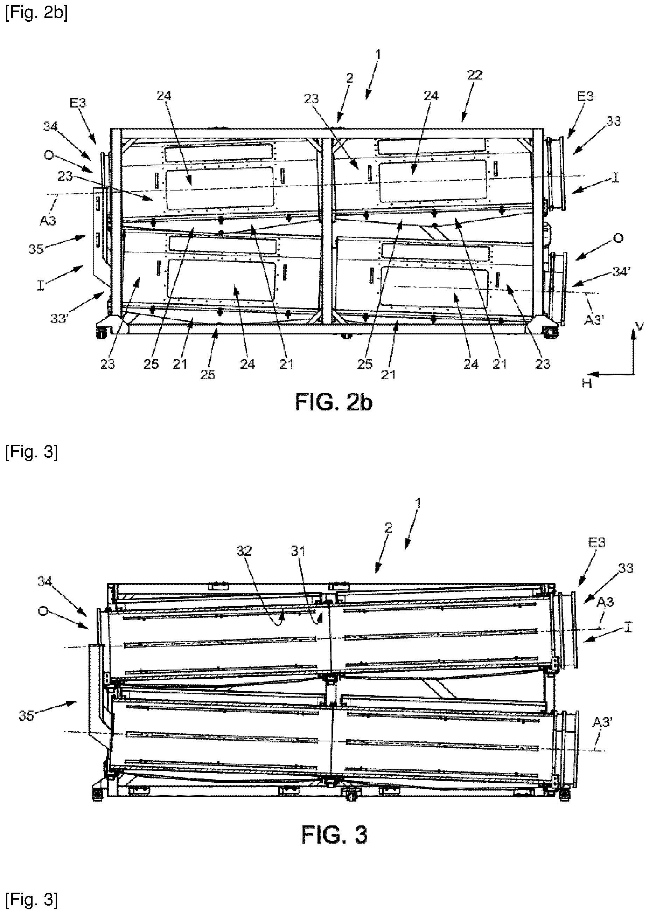

   [Fig. 2b] montre une vue de côté du dispositif de la figure 1.
**Fig. 3**
   [Fig. 3] montre une vue en coupe selon la ligne III-III de la figure 2a du dispositif de la figure 1.
**Fig. 4**

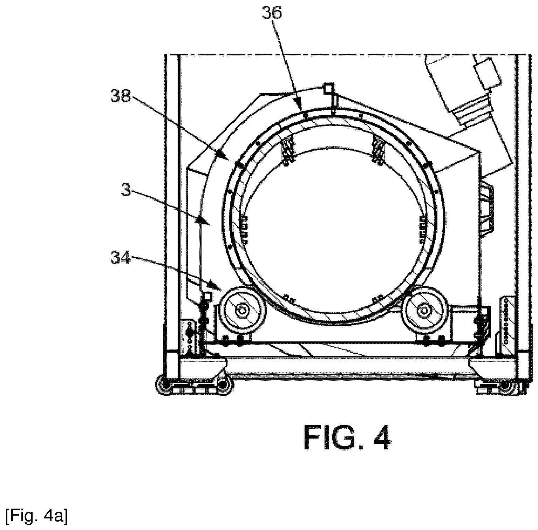

   [Fig. 4] montre une vue de détail en coupe selon la ligne IV-IV de la figure 2a du dispositif de la figure 1.
**Fig. 5**

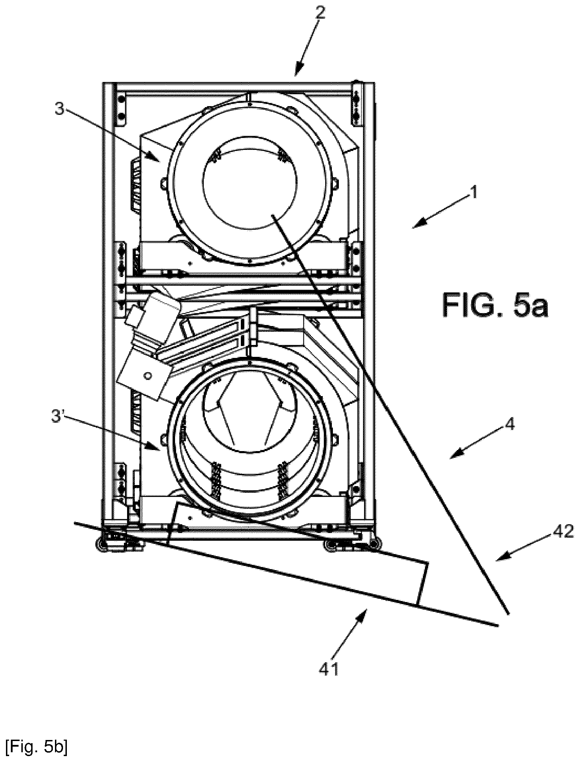

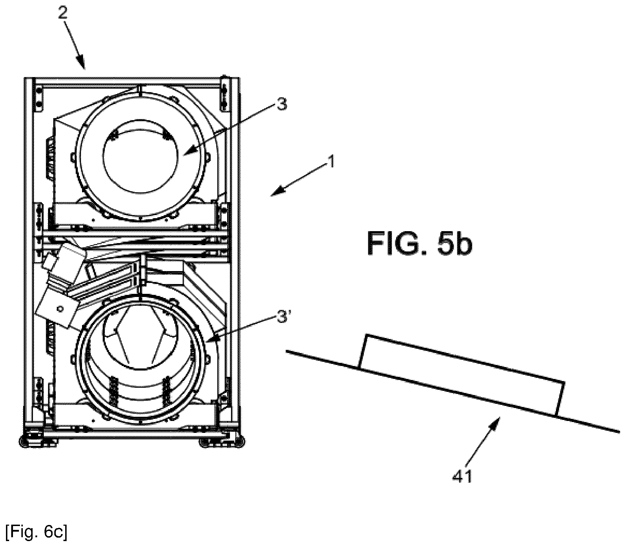

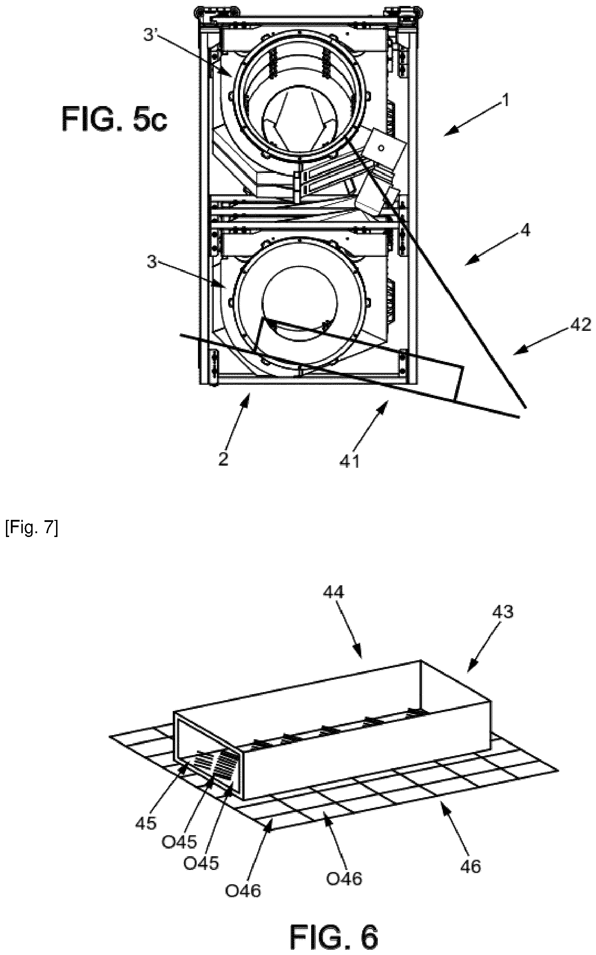

   [Fig. 5] montre une vue de face du dispositif de la figure 1, avec le circuit de recirculation et de tri, selon un première alternative, illustré schématiquement.
**Fig. 6**
   [Fig. 6] montre une vue en perspective du compartiment de réception vibrant d'un dispositif de séparation selon un mode de réalisation conforme à l'invention.
**Fig. 7**

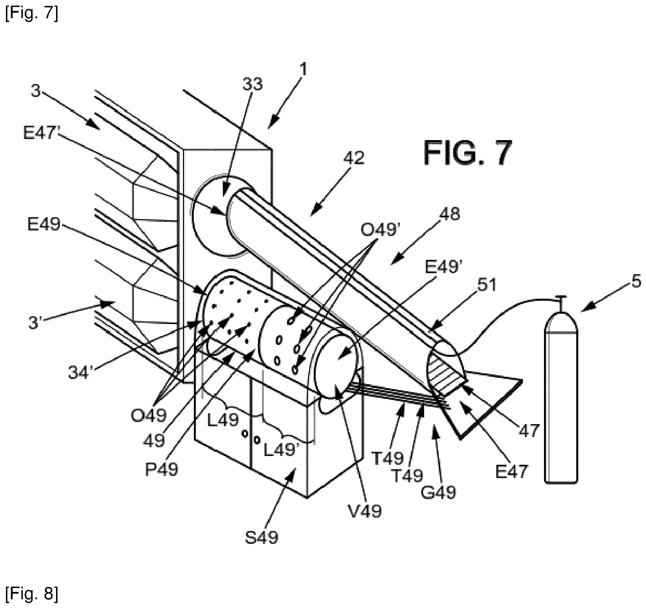

   [Fig. 7] montre une vue schématique en perspective du circuit de recirculation, selon une deuxième alternative.
**Fig. 8**

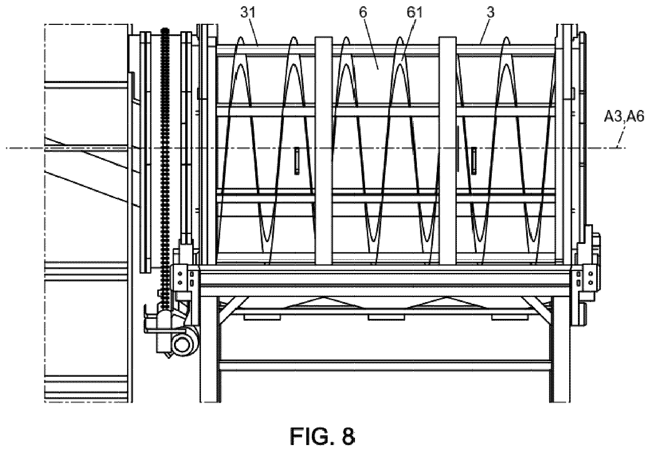

   [Fig. 8] montre une vue de détail en coupe verticale d'un dispositif selon un mode de réalisation conforme à l'invention.

### Description des modes de réalisation

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

Dans l'ensemble de la présente demande, l'Horizontale et la Verticale désignent les directions horizontales et verticales de l'espace, comme visible notamment sur les figures 1 et 2b.

L'invention concerne un procédé de séparation des trichomes de plantes de chanvre comprenant les étapes :
a) fourniture d'un dispositif 1 de séparation des trichomes de plantes de chanvre comprenant :
   - un bâti 2,
   - au moins un tambour 3 monté rotatif par rapport au bâti 2 autour de son axe longitudinal A3, la paroi périphérique 31 du tambour 3 étant constituée, au moins partiellement, d'un tamis 32, ledit tambour 3 comprenant une ouverture 33, 34 au niveau de chacune de ses extrémités longitudinales E3 matérialisant respectivement une entrée I et une sortie O pour la matière circulant dans ledit tambour 3, le tambour 3 étant disposé sur le bâti 2 de sorte que son axe longitudinal A3 soit, selon une première alternative : incliné par rapport à l'Horizontale H avec un angle non nul, de telle sorte que l'ouverture d'entrée 33 soit située au-dessus de l'ouverture de sortie 34 selon la Verticale V, ou selon une deuxième alternative : incliné sensiblement selon l'Horizontale H, de telle sorte que l'ouverture d'entrée 33 soit située sensiblement au même niveau que l'ouverture de sortie 34 selon la verticale V,
   - un circuit de recirculation et de tri 4, configuré pour recevoir de la matière en sortie O du tambour 3, la trier puis l'amener, au moins en partie, en entrée I du tambour 3,
   - au moins un compartiment de réception 21 des trichomes configuré de sorte à réceptionner les trichomes passés à travers le tamis 32 de la paroi périphérique 31 du tambour 3, après leur séparation du reste de la plante de chanvre dans ledit tambour 3 et à permettre leur évacuation du dispositif 1 de séparation par l'intermédiaire de moyens d'évacuation,
b) mise en rotation du tambour 3 par rapport au bâti 2 autour de son axe longitudinal A3,
c) insertion de pellets de glace carbonique dans le tambour 3 au travers de l'ouverture d'entrée 33 du tambour 3,
d) insertion de plantes de chanvre dans le tambour 3 au travers de l'ouverture d'entrée 33 du tambour 3,
e) réception de la matière au niveau de l'ouverture de sortie 34 du tambour 3 comprenant au moins des résidus de plantes de chanvre mélangés aux pellets de glace carbonique,
f) tri de la matière réceptionnée au niveau de l'ouverture de sortie 34 du tambour 3 afin de séparer les résidus de plantes de chanvre des pellets de glace carbonique puis amenée des pellets de glace carbonique au niveau de l'ouverture d'entrée du tambour 3,
g) évacuation des trichomes du au moins un compartiment de réception 21 des trichomes par l'intermédiaire des moyens d'évacuation.

Selon l'invention, les étapes b) à g) sont effectuées simultanément et en continu au cours du procédé.

Ainsi, le procédé selon l'invention est un procédé qui s'exécute en continu, dans lequel les matières sont introduites et extraites du tambour 3 du dispositif de séparation 1 de façon continue, et les trichomes des plantes de chanvre collectées dans le compartiment de réception 21 après leur passage au travers du tamis 32 de la paroi périphérique 31 du tambour 3, de manière continue et sans nécessiter l'arrêt de la mise en rotation du tambour 3.

En effet, grâce à l'inclinaison du tambour 3 par rapport à l'Horizontale, selon ladite première alternative du procédé, les matières introduites dans le tambour 3, au niveau de l'ouverture d'entrée 33, vont se déplacer dans le tambour 3 jusqu'à atteindre l'ouverture de sortie 34, sous l'effet combiné de la gravité et de la rotation du tambour 3 autour de son axe longitudinal A3.

Afin d'augmenter la capacité de production du procédé, le tambour 3 peut présenter une longueur importante, par exemple supérieure à 10 mètres, de préférence supérieure à 12 mètres, et/ou un diamètre important, par exemple supérieur à 1 mètre, de préférence supérieur à 1,20 mètre.

Également, afin de faciliter le déplacement des matières introduites dans le tambour 3 au cours de l'exécution du procédé selon l'invention, les matières peuvent être convoyées à l'intérieur du tambour 3 entre l'ouverture d'entrée 33 et l'ouverture de sortie 34 par un système de convoyage 6 de façon continue au cours du procédé selon l'invention, et notamment du procédé selon sa deuxième alternative, dans laquelle l'axe longitudinal A3 du tambour 3 est incliné sensiblement selon l'Horizontale H, de telle sorte que l'ouverture d'entrée 33 soit située sensiblement au même niveau que l'ouverture de sortie 34 selon la verticale V, et/ou lorsque le tambour 3 présente une longueur importante, par exemple supérieure à 10 mètres, de préférence supérieure à 12 mètres, et/ou lorsque le tambour 3 présente un diamètre important, par exemple supérieur à 1 mètre, de préférence supérieur à 1,20 mètre.

Les plantes de chanvre lors de leur parcours du tambour 3 vont être mises en mouvement et rouler contre la paroi périphérique 31 du tambour 3, ce qui a pour effet de détacher les trichomes du reste de la plante de chanvre. Les trichomes ainsi séparés de la plante de chanvre vont passer au travers des ouvertures du tamis 32 de la paroi périphérique 31 du tambour 3 afin d'être isolés des résidus de plantes de chanvre pour pouvoir être reçus ensuite dans le au moins un compartiment de réception 21 afin d'être récupérés.

Avec le procédé selon l'invention, il est donc possible de traiter une quantité de plantes de chanvre plus importante qu'avec les procédés de l'état de la technique, comme celui décrit dans le document EP 1 329 265 A2, qui nécessitent l'arrêt de la rotation du tambour pour pouvoir en extraire la matière ou l'introduire, ce qui permet, grâce au procédé selon l'invention, et selon les dimensions du tambour 3, de traiter une quantité importante de plantes de chanvre, par exemple plusieurs centaines de kilogrammes de plantes de chanvre, voire plusieurs tonnes par jour, ce qui rend le procédé compatible avec une application à l'échelle industrielle.

Également, et selon les constations de l'inventeur, le procédé selon l'invention permet avantageusement de réaliser une séparation des trichomes des plantes de chanvre sans traitement préalable des plantes de chanvre, par exemple pour les faire sécher ou pour ôter leurs tiges et/ou leur graines, ce qui permet de réaliser une séparation des trichomes des plantes de chanvre peu de temps, voire aussitôt après leur récolte, et notamment sans étape de séchage des plantes de chanvre préalable. Cela facilite la récupération de trichomes par rapport aux procédés de l'état de la technique prévoyant un traitement préalable des plantes de chanvre avant leur passage dans un dispositif de séparation des trichomes, comme par exemple celui décrit dans le document EP 1 329 265 A2, et réduit considérablement la durée entre la récolte des plantes de chanvre et la récupération des trichomes, avantageusement en passant de quelques jours à quelques minutes seulement.

Les pellets de glace introduits également dans le tambour 3 vont également parcourir ledit tambour 3 et venir heurter régulièrement les plantes de chanvre pour favoriser le détachement des trichomes, mais également refroidir les plantes de chanvre, avantageusement à une température négative, ce qui favorise également le détachement des trichomes des plantes de chanvre, en rendant les polis d'accroche des trichomes cassants.

L'emploi de pellets de glace carbonique pour refroidir les plantes de chanvre est particulièrement avantageux, en ce que le froid généré n'engendre pas d'humidité dans le tambour 3 qui pourrait nuire aux plantes de chanvre, et notamment aux trichomes, ou même au fonctionnement du dispositif de séparation 1.

Les pellets de glace carbonique peuvent également présenter une réserve de calories suffisante pour effectuer plusieurs passages au travers du tambour 3 depuis l'ouverture d'entrée 33 vers l'ouverture de sortie 34, afin d'effectuer le refroidissement d'un nombre important de plantes de chanvre, ce qui permet avantageusement de diminuer le coût de revient du procédé selon l'invention.

Avantageusement, afin d'augmenter considérablement l'effet de froid intense exercé par la glace carbonique sur les plantes de chanvre, le procédé selon l'invention peut être mis en œuvre dans un dispositif de séparation situé dans une atmosphère confinée, isolée de l'extérieur. De manière avantageuse également, ladite atmosphère confinée peut présenter une température négative.

Avantageusement, et afin de régler la vitesse d'avance de la matière dans le tambour 3 au cours du procédé selon l'invention, il peut être prévu une inclinaison réglable de l'axe longitudinal A3 du tambour 3 par rapport à l'Horizontale, par l'intermédiaire de moyens de réglage de l'inclinaison (non représentés). L'angle d'inclinaison entre l'axe longitudinal A3 du tambour 3 peut, comme visible plus particulièrement sur les exemples de réalisation des figures 1 à 3, être compris entre 10° et 30°.

L'inclinaison de l'axe longitudinal A3 du tambour 3 par rapport à l'Horizontale peut être réglée, avantageusement en combinaison avec le réglage de la vitesse de rotation du tambour 3 autour de son axe longitudinal A3, de sorte que les plantes de chanvre parcourent une distance de friction contre la paroi périphérique 31 du tambour 3 supérieure à 100 mètres, par exemple sensiblement égale à 125 mètres, ce qui permet d'assurer une séparation complète des trichomes des plantes de chanvre.

Avantageusement, la vitesse de rotation du tambour 3 autour de son axe longitudinal peut être prévue strictement inférieure à la vitesse de centrifugation de la matière dans le tambour 3, ce qui permet à la matière de rester constamment mobile dans le tambour 3 au cours du procédé, sans se retrouver plaquée contre la paroi périphérique 31 du tambour 3, ce qui pourrait nuire au détachement des trichomes des plantes de chanvre.

Comme visible sur les exemples de réalisation des figures 1 à 3, le bâti 2 peut comporter un châssis de support 22, constitué d'une pluralité de profilés métalliques assemblés entre eux, et configuré pour assurer le support du dispositif 1 de séparation.

Le bâti 2 peut également comporter un carter 23, avantageusement en plusieurs parties, configuré pour entourer le tambour 3, de sorte à l'isoler du milieu environnant. Au moins un volet 24 peut être prévu dans ledit carter 23 de sorte à fournir un accès au tambour 3 depuis l'extérieur du carter 23.

Le tamis 32 peut être par exemple un tamis métallique avec des ouvertures présentant une surface de l'ordre de plusieurs dizaines de micromètres carrés. La largeur des ouvertures peut notamment être sensiblement égale à 140 µm, à plus ou moins 30% près.

Selon un mode de réalisation, le dispositif de séparation 1 comprend en outre des moyens de mise en vibration de la paroi périphérique 31 du tambour 3, configurés pour mettre en vibration la paroi périphérique 31 du tambour 3 de façon continue au cours des étapes b) à g) du procédé.

De façon continue au cours du procédé, le tamis 32 de la paroi périphérique 31 du tambour 3 peut ainsi être décolmaté par la mise en vibration de la paroi périphérique 31 du tambour 3.

Grâce à cette disposition avantageuse de l'invention, on assure le décolmatage en continu du tamis 32 au cours du procédé selon l'invention, ce qui évite que le tamis 32 ne s'encrasse et que ses ouvertures ne se bouchent au cours du procédé selon l'invention.

En effet, et selon les constations de l'inventeur, une simple mise en vibration de la paroi périphérique 31 du tambour 3 permet d'assurer le décolmatage du tamis 32, sans nécessiter d'action supplémentaire.

Cela évite donc de devoir interrompre le procédé pour procéder au décolmatage du tamis 32 de la paroi périphérique 31 du tambour 3, ce qui permet d'améliorer le rendement du procédé et d'augmenter sa capacité de production.

Selon un mode de réalisation, les moyens de mise en vibration comprennent des billes de plastique configurées de sorte à venir percuter la paroi périphérique 31 du tambour 3 au cours de sa rotation autour de son axe longitudinal A3 afin d'entraîner sa mise en vibration. Ces billes assurent encore par leur action mécanique avec le chanvre la séparation des trichomes de la plante de chanvre.

Le procédé peut en outre comprendre une étape b'), simultanée avec les étapes b) à g) et continue au cours du procédé, d'introduction de billes de plastique au dans le tambour 3 travers de l'ouverture d'entrée 33 du tambour 3.

De façon avantageuse :
- au cours de l'étape e) les billes peuvent être réceptionnées au niveau de l'ouverture de sortie 34 du tambour 3 mélangées avec les résidus de plantes de chanvre et les pellets de glace carbonique, et
- au cours de l'étape f) les billes peuvent être séparées des résidus de plantes de chanvre et amenées au niveau de l'ouverture d'entrée 33 du tambour 3.

Les billes peuvent par exemple être des billes en plastique aux normes alimentaires, et notamment en polyoxyméthylène (POM). Leur diamètre peut avantageusement être compris entre 20 mm et 30 mm et présenter un poids compris entre 4 g et 7 g, afin de permettre une vibration suffisante de la paroi périphérique 31 du tambour 3 au cours du procédé, tout en ayant un encombrement réduit, ne nuisant pas à la circulation des plantes de chanvre dans le tambour 3.

Également, les pellets de glace carbonique, lorsque présents à l'intérieur du tambour 3, peuvent, au moins en partie, constituer lesdits moyens de mise en vibration de la paroi périphérique 31 du tambour 3.

Cela permet d'optimiser l'emploi de pellets de glace carbonique, qui en plus d'avoir pour fonction de refroidir les plantes de chanvre à l'intérieur du tambour 3, ont pour fonction de faire vibrer la paroi périphérique du tambour 3, ce qui permet éventuellement de diminuer le nombre de billes en plastique à introduire dans le tambour 3, et donc de diminuer le coût de revient du procédé selon l'invention.

Selon un mode de réalisation, les pellets de glace carbonique possèdent une température comprise entre -50°C et -90°C, de préférence entre -60°C et - 80°C, lorsque introduits dans le tambour 3 au travers de l'ouverture d'entrée 33 du tambour 3 au cours de l'étape c).

Avec une telle température des pellets de glace carbonique il est possible de refroidir les plantes de chanvre à une température négative, comme décrit plus haut, et également de pouvoir employer les mêmes pellets de glace carbonique pour plusieurs passages au travers du tambour 3, après leur tri au cours de l'étape f).

Selon un mode de réalisation, le dispositif de séparation 1 comporte deux tambours 3, 3' positionnés l'un au-dessus de l'autre selon la Verticale avec un tambour supérieur 3 et un tambour inférieur 3', le dispositif 1 comprenant en outre une gouttière 35 de communication entre le tambour supérieur 3 et le tambour inférieur 3', configurée de sorte à acheminer la matière au niveau de l'ouverture de sortie 34 du tambour supérieur 3 vers l'ouverture d'entrée 33' du tambour inférieur 3'.

Les plantes de chanvre et les pellets de glace, voire éventuellement les billes de plastique, peuvent être insérés au travers de l'ouverture d'entrée 33 du tambour supérieur 3 au cours des étapes c) et d) et les résidus de plantes de chanvre mélangés aux pellets de glace carbonique, voire éventuellement les billes de plastique, peuvent être réceptionnés au niveau de l'ouverture de sortie du tambour inférieur au cours de l'étape e).

Naturellement, l'ensemble des dispositions décrites ci-dessus et ci-dessous concernant le tambour 3 d'un dispositif 1 de séparation comprenant un unique tambour 3, s'appliquent aux deux tambours 3, 3' d'un dispositif 1 de séparation comprenant deux tambours 3, 3'. Dans une telle configuration à deux tambours 3' 3', l'étape f) de tri et de recyclage est opérée à la sortie O du tambour inférieur 3'.

Cette disposition avantageuse de l'invention permet d'augmenter, notamment sensiblement de doubler, la distance de friction parcourue par les plantes de chanvre contre la paroi périphérique 31 du tambour 3, sans augmenter la longueur du tambour 3, ce qui permet d'améliorer la séparation des trichomes des plantes de chanvre obtenue grâce au procédé selon l'invention, et sans augmenter l'encombrement longitudinal (selon l'Horizontale en position normale d'utilisation) du dispositif 1 de séparation, qui nuirait à la transportabilité du dispositif 1 de séparation, ce qui est désavantageux lorsque l'on souhaite pouvoir déplacer aisément le dispositif de séparation 1 afin de pouvoir exécuter le procédé selon l'invention en différentes localisations, et notamment à proximité de différents champs de plantes de chanvre, pour diminuer la durée entre la récolte des plantes de chanvre et la récupération des trichomes.

L'encombrement vertical du dispositif de séparation 1 (selon la Verticale en position normale d'utilisation) est lui augmenté mais reste bien inférieur à l'encombrement longitudinal, comme visible plus particulièrement sur les exemples de réalisation des figures 1 à 3.

Les tambours supérieur 3 et inférieur 3' peuvent être mis en rotation autour de leurs axes longitudinaux A3, A3' respectifs à une vitesse de rotation sensiblement identique, ou alors à une vitesse de rotation différente.

Comme visible sur les exemples de réalisation des figures 1 à 3, la gouttière 35 peut comporter deux plaques positionnées de part et d'autre de l'ouverture de sortie 34 du tambour supérieur 3 et de l'ouverture d'entrée 33' du tambour inférieur 3', configurées de sorte à guider la matière en l'empêchant de s'échapper du châssis 22, lorsqu'elle se trouve en dehors des tambours 3, 3'.

Avantageusement, et comme visible sur l'exemple de réalisation de la figure 1, l'ouverture d'entrée 33' du tambour inférieur 3' peut présenter une surface d'ouverture strictement inférieure à la surface d'ouverture de l'ouverture de sortie 34 du tambour supérieur 3, également afin d'empêcher la matière de s'échapper, au niveau de ladite ouverture d'entrée 33' du tambour inférieur 3'.

De façon plus générale, l'ouverture d'entrée 33, 33' d'un tambour 3, 3' peut présenter une surface d'ouverture strictement inférieure à la surface d'ouverture de l'ouverture de sortie 34, 34' d'un tambour 3, 3', afin d'empêcher la matière de s'échapper au cours de son introduction dans le tambour.

Selon un mode de réalisation, préalablement à l'étape d) d'insertion de plantes de chanvre dans le tambour 3 au travers de l'ouverture d'entrée 33 du tambour 3, les plantes de chanvre sont convoyées et refroidies simultanément préalablement à leur introduction au travers de l'ouverture d'entrée 33 du tambour 3 au cours de ladite étape d).

Grâce à cette disposition avantageuse de l'invention, les plantes de chanvre présentent une température inférieure à la température ambiante lors de leur entrée dans le tambour 3, et notamment une température négative, et sont donc congelés dès leur entrée dans le tambour 3, ce qui permet de limiter la quantité de pellets de glace carbonique à introduire dans le tambour 3 au cours du procédé selon l'invention, et donc de réduire sensiblement son coût de revient.

L'invention concerne également un dispositif de séparation 1 des trichomes de plantes de chanvre configuré pour permettre la mise en œuvre du procédé selon l'un des modes de réalisation décrit précédemment, comprenant :
- un bâti 2,
- au moins un tambour 3 monté rotatif par rapport au bâti 2 autour de son axe longitudinal A3, la paroi périphérique 31 du tambour étant constituée, au moins partiellement, d'un tamis 32 ledit tambour 3 comprenant une ouverture 33, 34 au niveau de chacune de ses extrémités longitudinales matérialisant respectivement une entrée I et une sortie O pour la matière circulant dans ledit tambour 3, le tambour 3 étant disposé sur le bâti 2 de sorte que son axe longitudinal A3 soit, selon une première alternative : incliné par rapport à l'Horizontale H selon avec un angle non nul, de telle sorte que l'ouverture d'entrée 33 est située au-dessus de l'ouverture de sortie 34 selon la Verticale V, ou selon une deuxième alternative : incliné sensiblement selon l'Horizontale H, de telle sorte que l'ouverture d'entrée 33 soit située sensiblement au même niveau que l'ouverture de sortie 34 selon la verticale V,
- un circuit de recirculation et de tri 4, configuré pour recevoir de la matière en sortie du tambour 3, la trier puis l'amener, au moins en partie, en entrée I du tambour 3,
- au moins un compartiment de réception 21 des trichomes configuré de sorte à réceptionner les trichomes passés à travers le tamis 32 de la paroi périphérique 31 du tambour 3, après leur séparation du reste de la plante de chanvre dans ledit tambour 3 et à permettre leur évacuation du dispositif de séparation 1 par l'intermédiaire de moyens d'évacuation.

L'ensemble des dispositions décrites ci-dessus concernant le dispositif 1 de séparation fourni au cours de l'étape a) du procédé selon l'invention s'appliquent également au dispositif de séparation 1 selon l'invention.

De même, l'ensemble des dispositions décrites concernant le dispositif 1 de séparation selon l'invention s'appliquent au dispositif de séparation 1 fourni au cours de l'étape a) du procédé selon l'invention.

Un tel dispositif de séparation 1 présente donc l'avantage de permettre le traitement d'une quantité importante de plantes de chanvre, en continu, ce qui le rend compatible avec une application à l'échelle industrielle.

Également, comme vu ci-dessus, un tel dispositif de séparation 1 présente un coût de fonctionnement réduit et permet également de réduire la durée entre la récolte des plantes de chanvre et la récupération des trichomes.

Selon un mode de réalisation, le dispositif de séparation 1 est configuré de sorte à mettre en œuvre le procédé selon un mode de réalisation, tel que décrit précédemment, dans lequel, de façon continue au cours du procédé, le tamis 32 de la paroi périphérique 31 du tambour 3 est décolmaté par la mise en vibration de la paroi périphérique 31 du tambour 3.

Le dispositif de séparation 1 peut à cet effet comprendre en outre des moyens de mise en vibration de la paroi périphérique 31 du tambour 3.

Comme décrit précédemment, de tels moyens de mise en vibration de la paroi périphérique 31 du tambour 3 peuvent comprendre des billes en plastique, telles que décrites précédemment, ou encore lesdits pellets de glace introduits dans le tambour 3 au cours du procédé selon l'invention mis en œuvre dans le dispositif de séparation 1 selon l'invention.

Selon un mode de réalisation, le dispositif 1 comporte au moins une piste de guidage 36 entourant la paroi périphérique 31 du tambour 3 et au moins un galet de guidage 37, monté rotatif sur le bâti 2, coopérant avec ladite piste de guidage 36 de sorte à assurer le guidage en rotation du tambour 3 par rapport au bâti 2 lors de sa rotation autour de son axe longitudinal A3.

Ladite piste de guidage 36 peut comporter au moins un plot 38 ménagé en saillie radiale vers l'extérieur du tambour 3, configuré de sorte que les contacts successifs entre le galet 37 et ledit plot 38 au cours de la rotation du tambour 3 autour de son axe longitudinal A3 entraînent la mise en vibration de la paroi périphérique 31 du tambour 3.

Ainsi, par le simple ajout dudit au moins un plot 38, la piste de guidage 36 et ledit au moins un galet de guidage 37 remplissent une fonction de moyens de mise en vibration de la paroi périphérique 31 du tambour 3, en plus de leur fonction de moyen de guidage en rotation du tambour 3 par rapport au bâti 2, lors de sa rotation autour de son axe longitudinal A3, ce qui permet de bénéficier de moyens de mise en vibration de conception particulièrement simple et de faible coût de revient, qui nécessitent peu de pièces supplémentaires.

Avantageusement, et comme visible plus particulièrement sur l'exemple de réalisation de la figure 4, il peut être prévu une pluralité de plots 38, répartis régulièrement autour de l'axe longitudinal A3 du tambour 3, le nombre et la répartition des plots 38 étant déterminés en fonction de la fréquence de vibration souhaitée pour la paroi périphérique 31 du tambour 3.

Il peut également être prévus au moins deux galets de guidage 37 coopérant avec ladite piste de guidage 36, à la fois pour améliorer le guidage en rotation du tambour 3 par rapport au bâti 2, et pour augmenter la fréquence de vibration de la paroi périphérique 31 du tambour 3.

Avantageusement, et comme visible plus particulièrement sur l'exemple de réalisation de la figure 2a, afin de s'assurer que la paroi périphérique 31 du tambour 3 est mise en vibration sur toute sa longueur, lors de la rotation du tambour 3 autour de son axe longitudinal A3, il peut être prévue une pluralité de pistes de guidage 36, munies chacune d'au moins un plot 38 et coopérant chacune avec au moins un galet de guidage 37, réparties sur la longueur du tambour 3, par exemple une à proximité de chaque extrémité longitudinale E3 du tambour 3 et une sensiblement à proximité du centre du tambour 3.

Selon un mode de réalisation, le au moins un compartiment de réception 21 des trichomes est solidaire du bâti 2 et est positionné en dessous du tambour 3 selon la Verticale V, en regard du tamis 32, ledit compartiment de réception 21 présentant une forme sensiblement pyramidale pointant vers le bas du tambour 3 selon un axe sensiblement vertical du bâti 2, une ouverture d'évacuation 25 étant prévue au niveau de la pointe de la pyramide formée par le compartiment de réception.

Ainsi, grâce à la disposition et à la forme d'un tel compartiment de réception 21, les trichomes ayant traversé le tamis 32 de la paroi périphérique 31 du tambour 3 s'écoulent par gravité jusqu'à la pointe du compartiment de réception 21, sensiblement en totalité, afin d'être évacués au niveau de ladite ouverture d'évacuation 25.

Comme visible sur l'exemple de réalisation de la figure 2a, le compartiment de réception 21 peut avantageusement former une pyramide non régulière, de sorte que sa pointe soit excentrée par rapport à l'axe longitudinal A3 du tambour 3, et de sorte que l'ouverture d'évacuation 25 se trouve décalée dudit axe longitudinal A3 afin d'améliorer son accessibilité.

Selon un mode de réalisation, au moins deux compartiments de réception 21 sont prévus, alignés selon l'axe longitudinal A3 du tambour 3, configurés de sorte à collecter les trichomes sur toute la longueur du tambour 3.

Les deux compartiments de réception 21 peuvent avantageusement être sensiblement identiques.

Avantageusement, et sans sortir du cadre de la présente invention, selon la longueur du tambour 3, il peut être prévu plus de deux compartiments de réception 21, alignés selon l'axe longitudinal A3 du tambour, afin de collecter les trichomes sur toute la longueur du tambour 3.

Selon un mode de réalisation, les moyens d'évacuation comprennent un système d'aspiration (non représenté) muni d'un tuyau d'aspiration dont une extrémité est fixée au niveau de l'ouverture d'évacuation 25 du compartiment de réception des trichomes 21.

Ainsi, grâce à l'emploi d'un système d'aspiration pour évacuer les trichomes hors du dispositif de séparation 1, il n'est pas nécessaire d'avoir un accès direct au dispositif de séparation 1, et notamment au tambour 3 ou au bâti 2, de sorte à récupérer les trichomes collectés dans le compartiment de réception 21.

Ceci peut être particulièrement avantageux lorsque ledit tambour 3 et ledit bâti 2 sont disposés dans une enceinte confinée renfermant une faible quantité d'oxygène, et dont l'accès présente un risque sanitaire pour un opérateur.

Également, l'emploi d'un système d'aspiration permet une évacuation en continue des trichomes collectés dans le compartiment de réception 21, sans arrêt de la mise en rotation du tambour 3 autour de son axe longitudinal A3, ou arrêt du procédé selon l'invention mis en œuvre dans le dispositif de séparation 1 selon l'invention.

Selon un mode de réalisation, ledit dispositif de séparation 1 comprend deux tambours 3, 3' positionnés l'un au-dessus de l'autre selon la Verticale V avec un tambour supérieur 3 et un tambour inférieur 3', le dispositif 1 comprenant en outre une gouttière de communication 35 entre le tambour supérieur 3 et le tambour inférieur 3', configurée de sorte à acheminer la matière au niveau de l'ouverture de sortie 34 du tambour supérieur 3 vers l'ouverture d'entrée 33 du tambour inférieur 3'.

Comme expliqué ci-dessus, concernant le procédé de séparation des trichomes de plantes de chanvre selon l'invention, cette disposition avantageuse de l'invention permet d'augmenter, sensiblement de doubler, la distance de friction parcourue par les plantes de chanvre contre la paroi périphérique 31 du tambour 3, sans augmenter la longueur du tambour 3, lors du fonctionnement du dispositif de séparation 1 selon l'invention, notamment lors de la mise en œuvre du procédé de séparation selon l'invention, ce qui permet d'améliorer la séparation des trichomes des plantes de chanvre, et sans augmenter l'encombrement longitudinal du dispositif 1 de séparation, qui nuirait à la transportabilité du dispositif 1 de séparation, ce qui est désavantageux lorsque l'on souhaite pouvoir déplacer aisément le dispositif 1 de séparation afin de pouvoir l'utiliser en différentes localisations, et notamment à proximité de différents champs de plantes de chanvre, pour diminuer la durée entre la récolte des plantes de chanvre et la récupération des trichomes.

L'encombrement vertical du dispositif 1 de séparation est lui augmenté mais reste bien inférieur à l'encombrement longitudinal, comme visible plus particulièrement sur les exemples de réalisation des figures 1 à 3.

Comme également expliqué ci-dessus, les tambours supérieur 3 et inférieur 3' peuvent être mis en rotation autour de leurs axes longitudinaux 3, A3' respectifs à une vitesse de rotation sensiblement identique, ou alors à une vitesse de rotation différente.

A cet effet, les tambours 3, 3' peuvent être mis en rotation par un moyen de motorisation distinct, ce qui facilite le pilotage de la vitesse de chacun des tambours 3, 3', en réduisant le coût de revient du dispositif de séparation 1, en évitant l'emploi de moyens de synchronisation complexes et coûteux.

Également, et selon la vitesse de déplacement de la matière souhaitée dans chaque tambour 3, 3', l'inclinaison du tambour supérieur 3 par rapport à l'Horizontale peut être sensiblement identique à l'inclinaison du tambour inférieur 3' par rapport à l'Horizontale, ou encore ces inclinaisons peuvent être différentes.

A cet effet, les moyens de réglage de l'inclinaison du tambour supérieur 3 par rapport à l'Horizontale peut être distinct des moyens de réglage de l'inclinaison du tambour inférieur 3' par rapport à l'Horizontale, ou en alternative il peut être prévu des moyens communs de réglage de l'inclinaison du tambour supérieur 3 et du tambour inférieur 3' par rapport à l'Horizontale.

Selon un mode de réalisation, le bâti 2 et ledit au moins un tambour 3 sont configurés de sorte à être disposés intégralement sur une remorque.

Avantageusement, et afin de pouvoir augmenter considérablement l'effet de froid intense exercé par la glace carbonique sur les plantes de chanvre, le dispositif de séparation 1 en entier, ou au moins le bâti 2 et ledit au moins un tambour 3, peuvent être configurés pour être disposés dans une remorque confinée, isolée de l'extérieur, et avantageusement une remorque frigorifique, configurée pour avoir une atmosphère interne présentant une température négative.

Ainsi, grâce à cette disposition avantageuse de l'invention, le bâti 2 et ledit au moins un tambour 3 peuvent être disposés sur une remorque, par exemple sur une remorque d'un véhicule de type poids lourd, et en particulier avec une remorque fermée, ce qui permet de pouvoir transporter aisément le dispositif de séparation 1 selon l'invention, et notamment au plus près du lieu de récolte des plantes de chanvre, ce qui permet encore de réduire la durée entre la récolte des plantes de chanvre et la récupération des trichomes.

De préférence, la remorque peut être une remorque frigorifique, avec une atmosphère intérieure présentant une température négative, ce qui permet d'augmenter considérablement l'effet de froid intense exercé par la glace carbonique sur les plantes de chanvre reçues dans le dispositif de séparation 1 selon l'invention.

Les autres éléments du dispositif de séparation 1, et notamment le circuit de recirculation et de tri 4 peuvent être configurés pour être positionnés, au moins partiellement en dehors de la remorque, au moins au cours du fonctionnement du dispositif de séparation 1. Ils peuvent en revanche être configurés pour être disposés intégralement sur ladite remorque lors du transport du dispositif de séparation 1, ce qui facilite le transport de l'ensemble des éléments du dispositif de séparation 1.

Le circuit de recirculation et de tri 4 peut avantageusement être prévu amovible du bâti 2 et du tambour 3 du dispositif de séparation 1 selon l'invention.

Selon un mode de réalisation, le circuit de recirculation et de tri 4 comporte :
- un système de réception et de tri 41, disposé au niveau de l'ouverture de sortie 34 du tambour 3, configuré de sorte à réceptionner la matière en sortie O du tambour 3 et à séparer les résidus de plantes de chanvre mélangés aux pellets de glace carbonique, et éventuellement aux billes de plastique,
- un moyen de convoyage 42 reliant ledit système de réception et de tri à l'ouverture d'entrée 33 du tambour 3 et configuré de sorte à convoyer les pellets de glace carbonique, et éventuellement les billes de plastique, après leur séparation des résidus de plante de chanvre au niveau du système de réception et de tri 41, jusqu'à l'ouverture d'entrée 33 du tambour 3,
- des moyens d'évacuation des résidus de plante de chanvre, après leur séparation des pellets de glace carbonique, et éventuellement des billes de plastique, au niveau du système de réception et de tri 41.

Ainsi, grâce à un tel circuit de recirculation et de tri 4, les matières en sortie O du tambour 3 peuvent être automatiquement triées de sorte à séparer les résidus de plantes de chanvre des pellets de glace carbonique, et éventuellement des billes de plastique, en vue de leur évacuation du dispositif de séparation 1, respectivement leur réintroduction dans le tambour 3, de sorte à assurer le fonctionnement en continu du dispositif de séparation 1 selon l'invention, et notamment pour l'exécution du procédé selon l'invention.

Le moyen de convoyage 42, représenté schématiquement à la figure 5 et à la figure 6 peut par exemple comprendre un ou plusieurs tapis roulants, ou tout autre moyen de convoyage connu de l'Homme du métier.

Comme expliqué ci-dessus, le circuit de recirculation et de tri 4 peut être prévu structurellement indépendant, et amovible du bâti 2 et du tambour 3 du dispositif de séparation 1, notamment afin, de faciliter le transport du dispositif de séparation 1, qui peut prévoir le transport séparé des différents éléments du dispositif de séparation 1, le bâti 2 et le tambour 3 formant un premier ensemble, et le circuit de recirculation et de tri 4 formant un deuxième ensemble, chacun de ces ensembles étant transporté, et éventuellement stocké séparément.

Après leur évacuation grâce aux moyens d'évacuation des résidus de plante de chanvre, les résidus de plante de chanvre peuvent être réemployés pour être valorisés, par exemple pour fabriquer de l'huile essentielle de chanvre.

Selon un mode de réalisation, notamment afin de permettre la mise en œuvre du procédé selon l'invention dans lequel, préalablement à l'étape d) d'insertion de plantes de chanvre dans le tambour 3 au travers de l'ouverture d'entrée 33 du tambour 3, les plantes de chanvre sont convoyées et refroidies simultanément préalablement à leur introduction au travers de l'ouverture d'entrée 33 du tambour 3 au cours de ladite étape d), le moyen de convoyage 42 peut, comme visible sur l'exemple de réalisation de la figure 7, comporter un tapis roulant 47 présentant une première extrémité longitudinale E47 et une deuxième extrémité longitudinale E47', avec :
- ladite première extrémité longitudinale E47 reliée audit système de réception et de tri 41, et
- ladite deuxième extrémité longitudinale E47' positionnée au niveau de l'ouverture d'entrée 33 du tambour 3.

Ledit tapis roulant 47 peut alors être logé, au moins sur toute sa longueur entre ladite première extrémité longitudinale E47 et ladite deuxième extrémité longitudinale E47', intégralement dans un carter 48.

Un système de refroidissement 5 peut alors être prévu, configuré de sorte à maintenir la température négative à l'intérieur du carter 48.

Comme visible sur l'exemple de réalisation de la figure 7, le système de refroidissement 5 peut être un système de refroidissement par gaz frigorifique, par exemple de l'azote liquide, avec une conduite 51, parcourant avantageusement ledit carter 48 sur toute la longueur du tapis roulant 47, de sorte à refroidir l'intérieur dudit carter 48.

L'emploi d'un système de refroidissement 5 par gaz frigorifique permet d'assurer une surgélation rapide des plantes de chanvre, de sorte qu'il est certain que celles-ci possèdent une température négative à leur arrivée au niveau de l'ouverture d'entrée 33 du tambour 3.

Grâce à l'emploi d'un tel système de convoyage 42, les plantes de chanvre sont surgelées sur ledit tapis roulant 47, entre sa première extrémité longitudinale E47 et sa deuxième extrémité longitudinale E47' avant leur introduction dans le tambour 3, au travers de l'ouverture d'entrée 33.

Selon un mode de réalisation, ledit système de réception et de tri 41 comporte une virole 49 de forme sensiblement cylindrique, solidaire du tambour 3, et sensiblement alignée avec ledit tambour 3.

Ladite virole 49 peut comprendre une première extrémité longitudinale E49 au niveau de l'ouverture de sortie O34 du tambour 3, et une deuxième extrémité longitudinale E49' présentant une ouverture d'évacuation V49.

Ladite virole 49 peut présenter une paroi périphérique P49 perforée, avec :
- sur une première portion longitudinale L49 de la paroi périphérique P49, s'étendant depuis la première extrémité longitudinale E49 de la virole 49, une pluralités d'ouvertures O49 de diamètre compris entre 0,5 mm et 3 mm, de préférence entre 1,5 mm et 2,5 mm,
- sur une deuxième portion longitudinale L49' de la paroi périphérique P49), s'étendant depuis la première portion longitudinale L49 vers la deuxième extrémité longitudinale E49' de la virole 49, une pluralités d'ouvertures O49' de diamètre compris entre 3,5 mm et 7 mm, de préférence entre 4mm et 6mm.

Ledit système de réception et de tri 41 peut comprendre également une goulotte G49 inclinée, par exemple selon une pente comprise entre 10° et 45°, reliant l'ouverture d'évacuation V49 au moyen de convoyage 42, ladite goulotte G49 comprenant une pluralité de tiges T49, disposées sensiblement parallèlement entre elles et écartées les unes des autres, avec une distance de séparation entre deux tiges consécutives T49 sensiblement constante.

La largeur de la goulotte G49 peut être prévue au moins égale à 20 cm, afin de faciliter son débourrage.

Un moyen de vibration supplémentaire (non représenté), par exemple un vibreur à masselottes, peut être prévu afin de mettre en vibration ladite goulotte G49, et faciliter le passage des différents éléments entre les tiges T49.

Ainsi, à sa sortie du tambour 3, au travers de l'ouverture de sortie 34, la matière issue du tambour 3 comprenant des résidus de plantes de chanvre, desquelles ont été séparées les trichomes, des pellets de glace carbonique et éventuellement des billes plastique est triée de la façon suivante :
- les fleurs et/ou les feuilles présentes dans les résidus de plantes de chanvre sont évacuées par les ouvertures O49 de la première portion longitudinale L49,
- les graines présentes dans les résidus de plantes de chanvre sont évacuées par les ouvertures O49' de la deuxième portion longitudinale L49',
- les branches présentes dans les résidus de plantes de chanvre sont évacuées en passant entre les tiges T49 de la goulotte G49.

Ainsi, les pellets de glace carbonique et éventuellement les billes de plastique sont séparés des résidus de plante de chanvre lors de leur arrivée au niveau du système de convoyage 42 et peuvent être réintroduits dans le tambour 3.

Les graines, les feuilles, voire les branches issues des résidus de plante de chanvre, après leur passage dans ledit système de réception et de tri 41, peuvent être récupérées pour être valorisées ultérieurement.

Également, la longueur de la première portion longitudinale L49 peut être comprise entre 450 mm et 750 mm, de préférence entre 500 mm et 600 mm.

De même, la longueur de la deuxième portion longitudinale L49' peut être comprise entre 200 mm et 500 mm, de préférence entre 300 mm et 400 mm.

Il peut être prévu une troisième portion longitudinale (non représentée) s'étendant depuis la deuxième portion longitudinale L49' jusqu'à la deuxième extrémité longitudinale E49', sur laquelle la paroi périphérique P49 de la virole 49 n'est pas perforée.

Ladite virole 49 peut être reçue sur un support S49, présentant un ou plusieurs compartiments de réception permettant la récupération des graines ou feuilles séparées des résidus de plante de chanvre dans ladite virole 49, comme décrit précédemment.

Également, ledit support S49 peut être configuré de sorte à confiner la virole 49 afin de l'isoler, au moins partiellement, de l'extérieur.

Les tiges T49 de la goulotte G49 peuvent être des tiges métalliques, par exemple en acier inoxydable.

La distance séparant deux tiges T49 consécutives peut avantageusement être inférieure au diamètre des pellets de glace carbonique et/ou des billes en plastique des moyens de mise en vibration, par exemple comprise entre 4 mm et 7 mm, de préférence entre 5 mm et 6 mm.

Selon un mode de réalisation alternatif représenté par exemple sur les exemples de réalisation des figures 5 et 6, le système de réception et de tri 41 comporte un compartiment de réception 43 vibrant comprenant :
- un bac de réception 44 présentant une paroi inférieure 45 sensiblement plane avec une pluralité d'ouvertures O45 sensiblement rectilignes,
- une paroi de support 46 sensiblement plane avec une pluralité d'ouvertures O46 sensiblement rectilignes, recevant en appui plan la paroi inférieure 45 du bac de réception 44, la paroi inférieure 45 du bac de réception 44 étant mobile en translation par rapport à la paroi de support 46 selon le plan d'appui entre ladite paroi inférieure 45 du bac de réception 44 et la paroi de support 46, avec une course limitée.

La surface des ouvertures O45 de la paroi inférieure 45 du bac de réception 44 peut être strictement inférieure à la surface des ouvertures O46 de la paroi de support 46 et les ouvertures O45 de la paroi inférieure 45 du bac de réception 44 peuvent être inclinées par rapport aux ouvertures O46 de la paroi de support 46, selon un angle non nul, par exemple compris entre 10° et 60°.

Le compartiment de réception vibrant 43 peut être configuré de sorte que sa mise en vibration entraîne un déplacement relatif entre la paroi inférieure 45 du bac de réception 44 et la paroi de support 46 selon ledit plan d'appui de sorte à entraîner le passage au travers des ouvertures O45 de la paroi inférieure 45 du bac de réception 44 et des ouvertures O46 de la paroi de support 46 des pellets de glace carbonique, et éventuellement des billes de plastique en vue d'être convoyés jusqu'à l'ouverture d'entrée 33 du tambour 3 par l'intermédiaire du moyen de convoyage 42, tout en empêchant le passage des résidus de plante de chanvre au travers desdites ouvertures O45, O46, de sorte à les maintenir dans ledit bac de réception 44, en vue d'être évacués par l'intermédiaire desdits moyens d'évacuation des résidus de plante de chanvre.

Un tel compartiment de réception vibrant 43 est de conception particulièrement simple et de faible coût de revient, notamment en comparaison avec les tables vibrantes employées de façon classique en agriculture pour le tri de végétaux.

Notamment, comme visible sur l'exemple de réalisation de la figure 6, la paroi de support 46 peut être constituée d'une simple grille, notamment métallique.

Afin de faciliter le guidage de la matière dans le bac de réception 44, des déflecteurs (non représentés) peuvent être positionnés au-dessus dudit bac de réception 44.

Le dispositif de séparation 1 des trichomes de plantes de chanvre, peut être prévu comme un équipement mobile et être intégré à un véhicule, au moins ledit tambour 3 (simple, ou double avec son tambour supérieur 3 et tambour inférieur 3') étant par exemple intégré à la caisse d'un camion ou d'une remorque de camion.

De manière générale, et en particulier lorsque le ou les tambours 3, 3' sont intégrés dans une caisse de camion ou de remorque, les moyens d'évacuation des trichomes du au moins un compartiment de réception 21 peuvent être du type aérauliques, les trichomes étant évacués portés par un flux d'air, et notamment de sorte à permettre la mise en œuvre du procédé sans intervention humaine dans la caisse pour assurer la séparation des trichomes de plantes de chanvre.

Selon un mode de réalisation, le dispositif de séparation 1 est configuré de sorte à mettre en œuvre le procédé selon un mode de réalisation, tel que décrit précédemment, dans lequel, de façon continue au cours du procédé, les matières sont convoyées à l'intérieur du tambour 3 entre l'ouverture d'entrée 33 et l'ouverture de sortie 34.

Le dispositif de séparation 1 peut à cet effet comprendre en outre un système de convoyage 6.

Comme décrit précédemment, et comme visible sur l'exemple de réalisation de la figure 8, un tel système de convoyage 6 peut comprendre une paroi en forme d'hélice 61 d'axe A61 sensiblement confondu avec l'axe longitudinal A3 du tambour 3 et s'étendant en saillie depuis la paroi périphérique 31 du tambour 3 vers l'axe longitudinal A3 du tambour 3.

Un tel système de convoyage 6 fonctionne à la manière d'une vis d'Archimède, l'hélice étant entraînée en rotation autour de son axe A61 par la rotation du tambour 3 autour de son axe longitudinal A3, et permet d'assurer un convoyage des matières à l'intérieur du tambour 3 avec une avance sensiblement constante, et sans risquer qu'une partie desdites matières ne soient pas convoyées. De plus, il présente une conception simple et un coût de revient réduit.

L'emploi d'un système de convoyage 6 s'avère particulièrement avantageux dans le cas où l'axe longitudinal A3 du tambour 3 est inclinés sensiblement selon l'Horizontale H, et que les matières ne peuvent pas ou difficilement être convoyées à l'intérieur de celui-ci sous l'effet de la gravité seule, ou encore lorsque le tambour 3 présente une longueur importante, par exemple supérieure à 10 mètres, de préférence supérieure à 12 mètres, et/ou lorsque le tambour 3 présente un diamètre important, par exemple supérieur à 1 mètre, de préférence supérieur à 1,20 mètre.

Naturellement, d'autres modes de réalisation auraient pu être envisagés par l'Homme du métier sans pour autant sortir du cadre de l'invention définie par les revendications ci-après.

### Liste des signes de référence

- 1.: Dispositif de séparation
- 2.: Bâti
- 21.: Compartiment de réception
- 22.: Châssis de support
- 23.: Carter
- 24.: Volet
- 25.: Ouverture d'évacuation
- 3, 3'.: Tambour
- I.: Entrée
- O.: Sortie
- A3, A3'.: Axe longitudinal
- E3.: Extrémité longitudinale
- 31.: Paroi périphérique
- 32.: Tamis
- 33, 33'.: Ouverture d'entrée
- 34, 34'.: Ouverture de sortie
- 35.: Gouttière
- 36.: Piste de guidage
- 37.: Galet de guidage
- 38.: Plot
- 4.: Circuit de recirculation et de tri
- 41.: Système de réception et de tri
- 42.: Moyen de convoyage
- 43.: Compartiment de réception
- 44.: Bac de réception
- 45.: Paroi inférieure
- O45.: Ouverture
- 46.: Paroi de support
- 47.: Tapis roulant
- E47.: Première extrémité longitudinale
- E47'.: Deuxième extrémité longitudinale
- 48.: Carter
- 49.: Virole
- P49.: Paroi périphérique
- E49: Première extrémité longitudinale
- E49'.: Deuxième extrémité longitudinale
- L49.: Première portion longitudinale
- L49'.: Deuxième portion longitudinale
- O49, 049'.: Ouvertures
- G49.: Goulotte
- T49.: Tiges
- S49.: Support
- V49.: Ouverture d'évacuation
- 5.: Système de refroidissement
- 51.: ConduiteO46. Ouverture
- H.: Horizontale
- V.: Verticale

## Revendications

1. Procédé de séparation des trichomes de plantes de chanvre comprenant les étapes :
a) fourniture d'un dispositif de séparation (1) des trichomes de plantes de chanvre comprenant :
- un bâti (2),
- au moins un tambour (3) monté rotatif par rapport au bâti (2) autour de son axe longitudinal (A3), la paroi périphérique (31) du tambour (3) étant constituée, au moins partiellement, d'un tamis (32), ledit tambour (3) comprenant une ouverture (33, 34) au niveau de chacune de ses extrémités longitudinales (E3) matérialisant respectivement une entrée (I) et une sortie (O) pour la matière circulant dans ledit tambour (3), le tambour (3) étant disposé sur le bâti (2) de sorte que son axe longitudinal (A3) soit, selon une première alternative : incliné par rapport à l'Horizontale (H) avec un angle non nul, de telle sorte que l'ouverture d'entrée (33) soit située au-dessus de l'ouverture de sortie (34) selon la Verticale (V), ou selon une deuxième alternative : incliné sensiblement selon l'Horizontale (H), de telle sorte que l'ouverture d'entrée (33) soit située sensiblement au même niveau que l'ouverture de sortie (34) selon la verticale (V),
- un circuit de recirculation et de tri (4), configuré pour recevoir de la matière en sortie du tambour (3), la trier puis l'amener, au moins en partie, en entrée du tambour (3),
- au moins un compartiment de réception (21) des trichomes configuré de sorte à réceptionner les trichomes passés à travers le tamis (32) de la paroi périphérique (31) du tambour (3), après leur séparation du reste de la plante de chanvre dans ledit tambour (3) et à permettre leur évacuation du dispositif de séparation (1) par l'intermédiaire de moyens d'évacuation,
b) mise en rotation du tambour (3) par rapport au bâti (2) autour de son axe longitudinal (A3),
c) insertion de pellets de glace carbonique dans le tambour (3) au travers de l'ouverture d'entrée (33) du tambour (3),
d) insertion de plantes de chanvre dans le tambour (3) au travers de l'ouverture d'entrée (33) du tambour (3),
e) réception de la matière au niveau de l'ouverture de sortie (34) du tambour (3) comprenant au moins des résidus de plantes de chanvre mélangés aux pellets de glace carbonique,
f) tri de la matière réceptionnée au niveau de l'ouverture de sortie (34) du tambour (3) afin de séparer les résidus de plantes de chanvre des pellets de glace carbonique puis amenée des pellets de glace carbonique au niveau de l'ouverture d'entrée (33) du tambour (3),
g) évacuation des trichomes du au moins un compartiment de réception (21) des trichomes par l'intermédiaire des moyens d'évacuation,
dans lequel les étapes b) à g) sont effectuées simultanément et en continu au cours du procédé.

2. Procédé selon la revendication 1, dans lequel le dispositif de séparation (1) comprend en outre des moyens de mise en vibration de la paroi périphérique (31) du tambour (3), configurés pour mettre en vibration la paroi périphérique (31) du tambour (3) de façon continue au cours des étapes b) à g) du procédé,
et dans lequel, de façon continue au cours du procédé, le tamis (32) de la paroi périphérique (31) du tambour (3) est décolmaté par la mise en vibration de la paroi périphérique (31) du tambour (3).

3. Procédé selon la revendication 2, dans lequel les moyens de mise en vibration comprennent des billes de plastique configurées de sorte à venir percuter la paroi périphérique (31) du tambour (3) au cours de sa rotation autour de son axe longitudinal (A3) afin d'entraîner sa mise en vibration,
le procédé comprenant en outre une étape b'), simultanée avec les étapes b) à g) et continue au cours du procédé, d'introduction de billes de plastique dans le tambour (3) au travers de l'ouverture d'entrée (33) du tambour (3),
et dans lequel :
- au cours de l'étape e) les billes sont réceptionnées au niveau de l'ouverture de sortie (34) du tambour (3) mélangées avec les résidus de plantes de chanvre et les pellets de glace carbonique, et
- au cours de l'étape f) les billes sont séparées des résidus de plantes de chanvre et amenées au niveau de l'ouverture d'entrée (33) du tambour (3).

4. Procédé selon l'une des revendications 1 à 3, dans lequel les pellets de glace carbonique possèdent une température comprise entre -50°C et - 90°C, de préférence entre -60°C et -80°C, lorsque introduits dans le tambour (3) au travers de l'ouverture d'entrée (33) du tambour (3) au cours de l'étape c).

5. Procédé selon l'une des revendications 1 à 4, dans lequel le dispositif de séparation (1) comporte deux tambours (3, 3') positionnés l'un au-dessus de l'autre selon la Verticale (V) avec un tambour supérieur (3) et un tambour inférieur (3'), le dispositif (1) comprenant en outre une gouttière de communication (35) entre le tambour supérieur (3) et le tambour inférieur (3'), configurée de sorte à acheminer la matière au niveau de l'ouverture de sortie (34) du tambour supérieur (3) vers l'ouverture d'entrée (33') du tambour inférieur (3'), dans lequel les plantes de chanvre et les pellets de glace sont insérés au travers de l'ouverture d'entrée (33) du tambour supérieur (3) au cours des étapes c) et d) et les résidus de plantes de chanvre mélangés aux pellets de glace carbonique sont réceptionnés au niveau de l'ouverture de sortie (34') du tambour inférieur (3') au cours de l'étape e).

6. Procédé selon l'une des revendications 1 à 5, dans lequel, préalablement à l'étape d) d'insertion de plantes de chanvre dans le tambour (3) au travers de l'ouverture d'entrée (33) du tambour (3), les plantes de chanvre sont convoyées et refroidies simultanément préalablement à leur introduction au travers de l'ouverture d'entrée (33) du tambour (3) au cours de ladite étape d).

7. Dispositif de séparation (1) des trichomes de plantes de chanvre configuré pour permettre la mise en œuvre du procédé selon l'une des revendications 1 à 6, comprenant :
- un bâti (2),
- au moins un tambour (3) monté rotatif par rapport au bâti autour de son axe longitudinal (A3), la paroi périphérique (31) du tambour (3) étant constituée, au moins partiellement, d'un tamis (32) ledit tambour (3) comprenant une ouverture (33, 34) au niveau de chacune de ses extrémités longitudinales (E3) matérialisant respectivement une entrée (I) et une sortie (O) pour la matière circulant dans ledit tambour (3), le tambour (3) étant disposé sur le bâti (2) de sorte que son axe longitudinal (A3) soit, selon une première alternative : incliné par rapport à l'Horizontale (H) avec un angle non nul, de telle sorte que l'ouverture d'entrée (33) est située au-dessus de l'ouverture de sortie (34) selon la Verticale (V), ou selon une deuxième alternative : incliné sensiblement selon l'Horizontale (H), de telle sorte que l'ouverture d'entrée (33) soit située sensiblement au même niveau que l'ouverture de sortie (34) selon la verticale (V),
- un circuit de recirculation et de tri (4), configuré pour recevoir de la matière en sortie (O) du tambour (3), la trier puis l'amener, au moins en partie, en entrée (I) du tambour (3),
- au moins un compartiment de réception (21) des trichomes configuré de sorte à réceptionner les trichomes passés à travers le tamis (32) de la paroi périphérique (31) du tambour (3), après leur séparation du reste de la plante de chanvre dans ledit tambour (3) et à permettre leur évacuation du dispositif de séparation (1) par l'intermédiaire de moyens d'évacuation.

8. Dispositif (1) selon la revendication 7, configuré de sorte à mettre en œuvre le procédé selon l'une des revendications 2 ou 3 comprenant en outre des moyens de mise en vibration de la paroi périphérique (31) du tambour (3).

9. Dispositif (1) selon la revendication 8 dans lequel le dispositif comporte au moins une piste de guidage (36) entourant la paroi périphérique (31) du tambour (3) et au moins un galet de guidage (37), monté rotatif sur le bâti (2), coopérant avec ladite piste de guidage (37) de sorte à assurer le guidage en rotation du tambour (3) par rapport au bâti (2) lors de sa rotation autour de son axe longitudinal (A3),
dans lequel ladite piste de guidage (36) comporte au moins un plot (38) ménagé en saillie radiale vers l'extérieur du tambour (3), configuré de sorte que les contacts successifs entre le galet (37) et ledit plot (38) au cours de la rotation du tambour (3) autour de son axe longitudinal (A3) entraînent la mise en vibration de la paroi périphérique (31) du tambour (3).

10. Dispositif selon l'une des revendications 7 à 9, dans lequel le au moins un compartiment de réception (21) des trichomes est solidaire du bâti (2) et est positionné en dessous du tambour (3) selon la Verticale (V), en regard du tamis (32), ledit compartiment de réception (21) présentant une forme sensiblement pyramidale pointant vers le bas du tambour (3) selon la Verticale (V), une ouverture d'évacuation (25) étant prévue au niveau de la pointe de la pyramide formée par le compartiment de réception (21).

11. Dispositif (1) selon la revendication 10 présentant au moins deux compartiments de réception (21), alignés selon l'axe longitudinal (A3) du tambour (3), configurés de sorte à collecter les trichomes sur toute la longueur du tambour (3).

12. Dispositif selon la revendication 10 ou 11, dans lequel les moyens d'évacuation comprennent un système d'aspiration muni d'un tuyau d'aspiration dont une extrémité est fixée au niveau de l'ouverture d'évacuation (25) du compartiment de réception (21) des trichomes.

13. Dispositif (1) selon l'une des revendications 7 à 12, comprenant deux tambours (3, 3') positionnés l'un au-dessus de l'autre selon la Verticale (V) avec un tambour supérieur (3) et un tambour inférieur (3'), le dispositif (1) comprenant en outre une gouttière de communication (35) entre le tambour supérieur (3) et le tambour inférieur (3'), configurée de sorte à acheminer la matière au niveau de l'ouverture de sortie (34) du tambour supérieur (3) vers l'ouverture d'entrée (33') du tambour inférieur (3').

14. Dispositif (1) selon l'une des revendications 7 à 13, dans lequel le bâti (2) et ledit au moins un tambour (3) sont configurés de sorte à être disposés intégralement sur une remorque.

15. Dispositif (1) selon l'une des revendications 7 à 14 dans lequel le circuit de recirculation et de tri (4) comporte :
- un système de réception et de tri (41), disposé au niveau de l'ouverture de sortie (34) du tambour (3), configuré de sorte à réceptionner la matière en sortie (O) du tambour (3) et à séparer les résidus de plantes de chanvre mélangés aux pellets de glace carbonique,
- un moyen de convoyage (42) reliant ledit système de réception et de tri (41) à l'ouverture d'entrée (33) du tambour (3) et configuré de sorte à convoyer les pellets de glace carbonique, après leur séparation des résidus de plante de chanvre au niveau du système de réception et de tri (41) jusqu'à l'ouverture d'entrée (33) du tambour (3),
- des moyens d'évacuation des résidus de plante de chanvre, après leur séparation des pellets de glace carbonique au niveau du système de réception et de tri (41).

16. Dispositif (1) selon la revendication 15 pour la mise en œuvre du procédé selon la revendication 6, dans lequel le moyen de convoyage (42) comporte un tapis roulant (47) présentant une première extrémité longitudinale (E47) et une deuxième extrémité longitudinale (E47'), avec :
- ladite première extrémité longitudinale (E47) reliée audit système de réception et de tri (41), et
- ladite deuxième extrémité longitudinale (E47') positionnée au niveau de l'ouverture d'entrée (33) du tambour (3),
dans lequel ledit tapis roulant (47) est logé, au moins sur toute sa longueur entre ladite première extrémité longitudinale (E47) et ladite deuxième extrémité longitudinale (E47'), intégralement dans un carter (48),
et dans lequel un système de refroidissement (5) est prévu, configuré de sorte à maintenir la température négative à l'intérieur du carter (48).

17. Dispositif (1) selon la revendication 15 ou 16, dans lequel ledit système de réception et de tri (41) comporte une virole (49) de forme sensiblement cylindrique, solidaire du tambour (3), et sensiblement alignée avec ledit tambour (3),
dans lequel ladite virole (49) comprend une première extrémité longitudinale (E49) au niveau de l'ouverture de sortie (O34) du tambour (3), et une deuxième extrémité longitudinale (E49') présentant une ouverture d'évacuation (V49),
et dans lequel ladite virole (49) présente une paroi périphérique (P49) perforée, avec :
- sur une première portion longitudinale (L49) de la paroi périphérique (P49), s'étendant depuis la première extrémité longitudinale (E49) de la virole (49), une pluralités d'ouvertures (O49) de diamètre compris entre 0,5 mm et 3 mm, de préférence entre 1,5 mm et 2,5 mm,
- sur une deuxième portion longitudinale (L49') de la paroi périphérique (P49), s'étendant depuis la première portion longitudinale (L49) vers la deuxième extrémité longitudinale (E49') de la virole (49), une pluralités d'ouvertures (O49') de diamètre compris entre 3,5 mm et 7 mm, de préférence entre 4mm et 6mm,
et dans lequel, ledit système de réception et de tri (41) comprend également une goulotte (G49) inclinée, reliant l'ouverture d'évacuation (V49) au moyen de convoyage (42), ladite goulotte (G49) comprenant une pluralité de tiges (T49), disposées sensiblement parallèlement entre elles et écartées les unes des autres, avec une distance de séparation entre deux tiges consécutives (T49) sensiblement constante.
